# EUROPEAN PATENT APPLICATION

(11) **EP 3 488 806 A1**
(43) Date of publication of application: **29.05.2019**
(21) Application number: 17203816.8
(22) Date of filing: 27.11.2017
(51) Int. Cl.: A61B 18/14, A61B 18/16

(54) **MULTIPOLAR RADIOFREQUENCY ABLATION DEVICE AND SYSTEM**

(71) Applicant: Universität Regensburg - Universitätsklinikum, 93053 Regensburg (DE)
(72) Inventor: Schicho, Andreas Johannes, 93055 Regensburg (DE)
(74) Representative: Lucke, Andreas

(57) **Abstract**

A radiofrequency ablation (RFA) device (10) comprising an inner shaft (12) and an outer shaft (14), wherein the inner shaft (12) is received inside the outer shaft (14). The outer shaft (14) is movable along a longitudinal direction with respect to the inner shaft (12). The outer shaft (14) comprises two or more spreadable parts (20). The inner shaft (12) has a cross-section perpendicular to the longitudinal direction that increases along the longitudinal direction, so that the two or more spreadable parts (20) can be made to progressively spread apart radially by moving the outer shaft (14) with respect to the inner shaft (12) along the longitudinal direction. The RFA device further comprises an electric current connector (28) electrically connected or connectable with an electric power source to connect at least one of the inner shaft (12) and the spreadable parts (20) electrically with the electric current connector (28).

## Description

### FIELD OF THE INVENTION

The present invention is in the field of medical electrical apparatuses. More particularly, the present invention relates to a radiofrequency ablation device with a novel configuration allowing for multipolar radiofrequency ablation of solid organs and tumours and a corresponding system.

### BACKGROUND OF THE INVENTION

Radiofrequency ablation (RFA) is a standardised technique for locally treating dysfunctional tissues in order to reduce their size or eliminate them, i.e. ablate them. A particularly important case of tissues on which RFA is applied is that of benign or malign tumours. The RFA technique is based on the fact that when an electric current is made to flow through a tissue, the tissue is locally damaged, in particular due to thermal heating around the entry point of the electric current into the tissue, i.e. around the position of an electrode.

RFA has the important advantages of allowing for a specific and localised treatment of the desired tissue without significant collateral damage. Further, RFA does not require a direct stimulation of nerves or muscles, which allows for its use without the need for general anaesthetic.

RFA is typically applied by incising a RFA device into the dysfunctional tissue through a small cut in the skin of the patient. Medical imaging techniques, like for example ultrasound, computer tomography or magnetic resonance imaging, can be used in parallel in order to ensure a precise positioning of the RFA device. The RFA device functions as an electrode through which the electrical current is made to flow through the tissue to an external electrode attached to the patient's body, for example to their thigh, buttock or back. Both electrodes are connected via electric wires to a power supply forming a closed circuit. The RFA device is the entry point of the electric current into the tissue around which the tissue is locally thermally ablated.

An ablation treatment of the target region of the patient's body over as much of the spatial extension of the dysfunctional tissue as possible is achieved by moving the RFA device within the tissue. Some of the existing RFA devices include a plurality of flexible ramifications that can branch off from the main body of the RFA device and can operate as secondary electrodes and hence contribute to a broader spatial coverage of the treated tissue. One such RFA device is described in US 2008/0154259 A1. Flexible ramifications are however unsuitable to treat hard tissues or tumours, like for example, osseous and dense muscular structures. Further, flexible ramifications provide optical obstacles that difficult a monitoring of the treatment by the parallel use of imaging techniques.

The size of a dysfunctional tissue region or tumour that can be treated with existing RFA devices is limited. The reason for this is that the tissue immediately surrounding the ablation electrode is exposed to high temperatures, which causes a high and effective destruction thereof but also results in a fast reduction of the electrical conductivity of the tissue, which negatively affects the subsequent flow of the electric current through the tissue and hence the efficiency of the ablation treatment. Big tumours and tumours having an irregular geometry can therefore only be treated partially. This is very unsatisfactory in medical terms, for a little amount of tumorous cells surviving the ablation treatment may suffice for triggering a new tumorous formation.

A further disadvantageous aspect of existing RFA devices derives from the fact that tumours are often located very close to critical anatomical structures, like for instance inside the liver close to big liver vessels or next to vital blood vessels, the lung or the heart. Incidental damage of these critical anatomical structures can result in severe injury or even in the death of the patient. However, existing RFA devices require the movement of the RFA device throughout the treated tissue all the way into the boundaries thereof, which may be critically close to one of the aforesaid anatomical structures.

There is hence a demand in the art for improved RFA devices and systems that allow for an efficient radiofrequency ablation treatment of dysfunctional tissues irrespectively of their size, hardness, geometry, and location in the patient's body.

### SUMMARY OF THE INVENTION

It is an object of the present invention to provide a radiofrequency ablation (RFA) device and system overcoming the technical disadvantages exposed above. This is achieved by a RFA device according to claim 1 and a RFA system according to claim 9. Preferable embodiments of the invention are captured by the dependent claims.

A radiofrequency ablation device according to the invention comprises an inner shaft, an outer shaft, and an electric current connector. The inner shaft extends along a longitudinal direction between a connection end and an ablation end. The outer shaft extends along the longitudinal direction and has a hollow lumen extending along the longitudinal direction. The inner shaft is received inside the hollow lumen. Thus both the inner shaft and the outer shaft extend along the longitudinal direction, which defines the direction along which the longest dimension, i.e. the length, of the RFA device extends. The outer shaft surrounds and encloses the inner shaft, which is at least partly received within the outer shaft.

The outer shaft is movable along the longitudinal direction with respect to the inner shaft. The outer shaft comprises a first portion and a second portion, wherein the second portion is closer to the ablation end than the first portion. The second portion of the outer shaft comprises two or more spreadable parts extending between the first portion of the outer shaft and a respective tip of the spreadable part.

The inner shaft has a cross-section perpendicular to the longitudinal direction that increases along the longitudinal direction towards the ablation end, so that the two or more spreadable parts can be made to progressively spread apart radially by moving the outer shaft with respect to the inner shaft along the longitudinal direction towards the ablation end. "Cross-section" refers herein to a surface perpendicular to the longitudinal direction that is enclosed by the outermost surface of the inner shaft irrespectively of whether the inner shaft is hollow, e.g. has a tube-like shape, or solid. When the spreadable parts of the outer shaft spread apart radially, they may spread away from an outermost surface of the inner shaft.

The electric current connector is electrically connected or connectable with an (outer) electric power source. At least one of the inner shaft and the spreadable parts is electrically connected with the electric current connector. The electric current connector may for example comprise a conductive connection port, terminal or plug and may be located adjacent to the outermost surface of the outer shaft. The electrical connection between at least one of the inner shaft and the spreadable parts and the electric current connector may be provided by internal connecting wires within the RFA device, by electrically conducting material strips or by the inner shaft and/or the outer shaft themselves when they are made of an electrically conductive material.

The electrical connection between the electric current connector and the at least one of the inner shaft and the spreadable parts may be such that an electrical current may enter the RFA device through the electrical current connector and propagate through the at least one of the inner shaft and the spreadable parts, through the body of the patient, and out of the body of the patient through a further electrode also incised into or attached to the body of the patient, wherein an electric potential between the RFA device and the further electrode drives the electric current. The further electrode may for example comprise a second RFA device or an outer electrode.

By moving the outer shaft with respect to the inner shaft along the longitudinal direction, a portion of the inner shaft that is received within the outer shaft can be selectively and continuously varied. However, the portion of the inner shaft that is received within the outer shaft can also be varied stepwise. The overall length of the RFA device may hence vary between a minimal length of the RFA device corresponding to the collapsed position and a maximal length corresponding to a situation in which the spreadable parts are spread apart and protrude from the ablation end. The RFA device may extend along a direction that deviates in part from a straight direction. In this case, the longitudinal direction may not be a straight direction.

The outer shaft may be in a collapsed position, in which the spreadable parts are aligned with the longitudinal direction so that the spreadable parts are flush with an outermost surface of the first portion of the outer shaft. In the collapsed position, the length of the RFA device may be minimal and the portion of the inner shaft received within the outer shaft may be maximal. In the collapsed position, a separation between the spreadable parts and a central axis of the outer shaft aligned with the longitudinal direction may be smaller than a separation between the spreadable parts and said central axis when the spreadable parts are spread apart. In the collapsed position, a separation between the spreadable parts and the aforesaid central axis of the outer shaft may be approximately the same at the tips of the spreadable parts and at a limiting region between the second portion and the first portion of the outer shaft. In other words, the spreadable parts may run substantially parallel to the longitudinal direction. In the collapsed position, the spreadable parts need not protrude along the longitudinal direction from the ablation end so that the ablation end of the inner shaft may form an end of the RFA device.

The outer shaft may comprise gaps between the spreadable parts, such that the two or more spreadable parts are separated from each other by two or more corresponding gaps formed in the outer shaft. Preferably, the number of gaps corresponds to the number of spreadable parts. The gaps may extend along the longitudinal direction within the second portion from the tips and for a length that may coincide with a length of the spreadable part along the longitudinal direction. In some embodiments, the gaps may, in the collapsed position, have the form of a straight slit. However, it is also possible that the gaps have a varying shape, and in particular a varying width.

The spreadable parts may be made of an elastic material, like for example a spring steel or an elastic plastic material. When the outer shaft is moved along the longitudinal direction towards the ablation end, due to the increasing cross-section of the inner shaft, its outermost surface exerts a radial force upon the spreadable parts that causes the spreadable parts to bend outwards and to thereby spread apart radially. In this situation, the tips of the spreadable parts may protrude from the ablation end of the inner shaft along the longitudinal direction, i.e. may be further away from the connection end of the inner shaft than the ablation end of the inner shaft.

The rigidity and the elasticity of the outer shaft may be chosen such as to allow for the aforesaid bending of the spreadable parts. In other words, the spreadable parts may be rigid enough for their tips to be displaced away and separated radially from the outermost surface of the inner shaft when the outer shaft is moved towards the ablation end. Further, the spreadable parts may be elastic enough to move back to the collapsed position and recover their original shape when the outer shaft is moved back towards the connection end and the radial forces exerted by the inner shaft on the spreadable parts disappear.

If the rigidity of the outer shaft were too low, the outer shaft could elastically adapt to the increasing cross-section of the inner shaft when moving along the longitudinal direction towards the ablation end, and the radial force exerted by the outermost surface of the inner shaft upon the spreadable parts would not cause the tips of the spreadable parts to spread apart and separate from the inner shaft. Further, the spreadable parts would be prone to breaking or irreparably deforming when operating in hard tissues like osseous tumours or dense muscular structures.

If the rigidity of the outer shaft were too high, the radial force exerted by the outermost surface of the inner shaft upon the spreadable parts could cause the spreadable parts to break or to be irreparably deformed, which would prevent them from recovering their original shape in the collapsed position. This would cause problems when moving the RFA device within the body of the patient and when extracting the RFA device from the body of the patient.

The RFA device according to the invention allows treating a greater tissue volume with a single incision compared to other RFA devices known in the art. The RFA device can be introduced into the body of the patient while being in the collapsed position. Once the ablation end of the inner shaft is located within the dysfunctional tissue or tumour to be ablated, the outer shaft can be moved with respect to the inner shaft along the longitudinal direction so that the spreadable parts spread apart radially. The RFA device may be moved and rotated within the tissue so as to cover a greater tissue volume. Thus, the RFA device of the invention allows applying the radioablation technique to tumours of greater size than those that were suitable for being treated in the prior art.

Further, the RFA device according to the invention makes it unnecessary to incise the RFA device into the dysfunctional tissue or tumour all the way to an outer boundary thereof that may be close to critical anatomical structures. Instead, the inner shaft can remain fixed to the tissue at a greater distance from the boundary of the treated tissue, which can be made accessible to the ablating electric current by the spreadable parts. The - less invasive - level of penetration of the spreadable parts into the tumour in a given direction can easily be controlled by carefully moving the outer shaft with respect to the inner shaft accordingly.

The RFA device of the invention allows configurations with a low number of spreadable parts, which reduces optical obstruction of the tissue for imaging and monitoring purposes. In addition, since the spreadable parts are made of a rigid material, the RFA device of the invention is suitable for treating tumours located in hard tissue regions of the patient's body that cannot be treated with prior art devices. The rigidity and solidity of the RFA device further eases the movement of the RFA device within the treated tissue, translations, rotations, and combinations thereof, and the removal of the RFA device from the patient's body once the ablation treatment is finished.

In preferred embodiments of the invention, two or more of the inner shaft and the spreadable parts may be electrically connected with the electric current connector. This allows creating an electric potential between the two or more of the inner shaft and the spreadable parts such that the RFA device may operate as a multi-electrode or multi-pole RFA device. An electric current may enter the RFA device through the electric current connector and circulate through one or more of the inner shaft and the spreadable parts acting as electrodes of one polarity, through the body of a patient, and through one or more of the inner shaft and the spreadable parts acting as electrodes of an opposite polarity out of the body of the patient through the electric current connector.

However, the two or more of the inner shaft and the spreadable parts may also be connected or connectable to a single electric potential, i.e. be used as electrodes of the same polarity. The RFA device according to the invention may allow selectively and/or alternatingly using each of the two or more of the inner shaft and the spreadable parts as electrodes. For example, each of the two or more of the inner shaft and the spreadable parts can be alternated in their use as electrodes for regular or irregular time periods. This way, an overheating of the tissue region surrounding a particular electrode and eventually causing a reduction of the conductivity of the tissue and hence of the ablation treatment can be avoided. Note that this advantageous effect is achieved irrespectively of whether the two or more of the inner shaft and the spreadable parts are connected or connectable to the same electric potential or to electric potentials of opposite polarities.

According to preferred embodiments of the invention, the inner shaft and each of the spreadable parts may be electrically connected with the electric current connector. This allows each of the inner shaft and the spreadable parts of the outer shaft to function as individual electrodes, which can preferably be operated mutually independently of each other. Since each of the inner shaft and the spreadable parts can act as independent electrodes, this effectively provides a three-dimensional distribution of the different electrodes and increases the tissue volume that can be treated.

In preferred embodiments of the invention, the inner shaft may comprise a first tip at the ablation end. The first tip of the inner shaft may then work as a spatially localized electrode through which the ablation electric current flowing through the inner shaft exits the inner shaft when an electric current flows through the inner shaft. This allows for an increased precision for a localised treatment of the tissue to be treated.

According to a preferred embodiment of the invention, the RFA device may further comprise a fluid connector connected or connectable with a fluid supply, for example a fluid pumping device. The inner shaft may comprise an inner fluid lumen fluidly connecting the ablation end with the fluid connector. Additionally or alternatively, one or more of the spreadable parts may comprise a spreadable part fluid lumen fluidly connecting the respective tip with the fluid connector and/or with the inner fluid lumen. The fluid connector, which may for example comprise a hose or the like, may allow a fluid from a fluid reservoir, for instance an infusion, to flow through the inner fluid lumen and/or through the spreadable part fluid lumens.

The fluid connector may for example comprise an intake/outtake in fluid communication with an inner fluid lumen of the inner shaft and/or with a spreadable part fluid lumen of one or more of the spreadable parts that fluidly connect the fluid connector with the ablation end and the tips of the spreadable parts respectively. An end of the inner fluid lumen next to the ablation end and/or an end of each of the spreadable part fluid lumens next to the respective tip may define openings in the inner shaft and the spreadable parts, respectively, and may operate as outtakes/intakes through which fluid can flow from the fluid supply and/or the fluid reservoir to the tissue being treated or vice versa. The inner fluid lumen and the one or more spreadable part fluid lumens may join to form a common lumen shared for a portion of their lengths so that a fluid connection between the fluid connector and the common lumen is shared by the inner fluid lumen and the spreadable part fluid lumens. The fluid connector can hence act as a fluid intake/outtake common to all of the inner fluid lumen and the one or more spreadable part fluid lumens.

In a preferred embodiment of the invention, the RFA device may further comprise one or more temperature sensors respectively provided at the inner shaft and/or at the spreadable parts. The temperature sensors allow the RFA device to obtain temperature information about a temperature of the inner shaft and/or of the spreadable parts and about a temperature of the regions of tissue around the inner shaft and/or the spreadable parts. This temperature information may be outputted to an external processing unit and may be used for example for controlling the alternate use of the inner shaft and the spreadable parts as ablation electrodes. The temperature sensors may be in communication with an external device configured to process information provided by the temperature sensors. A connection between the temperature sensors and such an external device may be provided through the electric current connector.

According to a preferred embodiment of the invention, the RFA device may further comprise a hollow insulating sleeve partly enclosing the inner shaft and the outer shaft, so that the inner shaft and the outer shaft may be partly received in the insulating sleeve. The hollow insulating sleeve may provide electrical and/or thermal isolation of a portion of the inner shaft and a portion of the outer shaft respectively received within the hollow insulating sleeve. The hollow insulating sleeve may comprise an electrically insulating plastic material, like for example a polyamide material, a polyethylene material, a polypropylene material or any combination thereof.

In preferred embodiments of the invention, the inner shaft may have a circular, a triangular or a polygonal cross-section. The outer shaft may also have a circular, a triangular or a polygonal cross-section. The number of the spreadable parts may be adapted to the geometry of the inner shaft, although different numbers of spreadable parts may be possible for each particular geometry of the inner shaft. In other words, the shape of the cross-section of the inner shaft need not correspond to the shape of the cross-section of the outer shaft and different combinations thereof are possible. The geometry of the inner shaft and/or the outer shaft need not be symmetric around the longitudinal direction, i.e. around a longitudinal axis of the RFA device. Further, the number of spreadable parts may, but need not correspond to a number of outermost surfaces of the inner shaft. For example, the inner shaft may have a quadrangular cross-section and four respective outermost surfaces extending along the longitudinal direction, i.e. perpendicular to the cross-section. In this case, the outer shaft may comprise four spreadable parts, each extending over one of the outermost surfaces of the inner shaft. However, the outer shaft may also comprise four spreadable parts if the inner shaft has a circular cross-section, and the outer shaft may comprise a number of spreadable parts different from four when the inner shaft has a quadrangular cross-section.

It is worth pointing out that, although reference is repeatedly made herein to regular geometries of the inner shaft and/or of the outer shaft, the RFA device, and in particular the inner shaft and/or the outer shaft may as well have irregular geometries. For example, the inner shaft and/or the spreadable parts may have irregular cross-sections with no well-defined symmetry axis.

According to a preferred embodiment of the invention, the inner shaft and/or the outer shaft may be made of carbon, of a metallic material, preferably of stainless steel, of titanium, or of a combination thereof, or of a plastic material, preferably of a polycarbonate resin, or of a thermoplastic elastomer, or of a combination thereof. The material composition of the inner shaft, the outer shaft, and all the remaining components of the RFA device may further be chosen such as to allow operating the RFA device in strong magnetic fields so that it may be used in combination with magnetic resonance imaging techniques. Further, the material composition of the inner shaft, the outer shaft, and one or more of the remaining components of the RFA device may be chosen such as to be compatible with a flow of acid fluids through lumens in the RFA device and/or able of being in contact with acids without suffering damage.

In some preferred embodiments of the invention, a length of the outer shaft along the longitudinal direction may exceed a length of the inner shaft along the longitudinal direction by between 10 mm and 80 mm, preferably by between 20 and 60 mm, more preferably by between 25 and 50 mm.

In some preferred embodiments of the invention, a length of the RFA device may be between 100 mm and 250 mm, preferably between 120 mm and 230 mm, more preferably by 140 and 210 mm.

According to a preferred embodiment of the invention, the RFA device may further comprise a control grip or handle operatively coupled with the inner shaft and the outer shaft, wherein the control grip is configured for controlling a movement of the outer shaft along the longitudinal direction with respect to the inner shaft. Thus, a human or a machine operator may easily control the RFA device, for example the relative movement of the outer shaft with respect to the inner shaft along the longitudinal direction, by operating the control grip. The control grip may be ergonomically designed and may be configured for allowing a human operator holding and operating the RFA device, for example, for moving and rotating the RFA device.

A further aspect of the invention relates to a radiofrequency ablation, RFA, system comprising a RFA device according to one of the embodiments of the invention described above, an electric power source connected to the electric current connector of the RFA device, and a control unit configured for controlling an electric current provided by the electric power source. The control unit allows the electric current provided by the electric power source being controlled by a human or a machine operator such as to provide the electric current desired or required for RFA treatment.

In preferred embodiments of the invention, two or more of the inner shaft and the spreadable parts of the RFA device are electrically connected with the electric current connector, and the control unit is further configured for controlling the electric current provided by the electric power source to circulate between the two or more of the inner shaft and the spreadable parts. The electric power source is then electrically connected between the two or more of the inner shaft and the spreadable parts thereby allowing forming a closed electric circuit through which an electric current generated by the electric power source can flow through one or more of the inner shaft and the spreadable parts acting as electrode(s) of one polarity, through the tissue being treated, and through different one or more of the inner shaft and the spreadable parts acting as electrode(s) of an opposite polarity.

According to a preferred embodiment of the invention, the RFA system further comprises at least one outer electrode, wherein the electric power source is further connected to the at least one outer electrode, and wherein the control unit is further configured for controlling the electric current provided by the electric power source to circulate between the at least one of the inner shaft and the spreadable parts and the at least one outer electrode. The at least one outer electrode may be suitable for being attached to the patient's body, for example to his or her back, thigh or buttocks.

The electric power source is then electrically connected between the electric current connector and the at least one outer electrode thereby allowing forming a closed electric circuit through which an electric current generated by the electric power source can flow through the at least one outer electrode, a tissue being treated, and the RFA device. The at least one outer electrode is suitable for being attached to the patient's body, for example to his or her back, thigh or buttocks.

In a preferred embodiment of the invention, the control unit may be configured for controlling a strength of the electric current provided by the electric power source, for example by means of a voltage or a voltage pattern applied to the selected one(s) of the inner shaft and the spreadable parts.

In other preferred embodiments of the invention, the control unit and the RFA device may be configured such that the control unit can selectively provide the electric current to one or more of the inner shaft and the spreadable parts. The control unit can then be used to select the inner shaft and/or one or more of the spreadable parts as an active electrode. However, it is also possible to apply voltages of different polarities to different ones of the inner shaft and one or more of the spreadable parts so that an electric current flows in between. This way, RFA treatment may be provided to a patient without the need of an outer electrode. The control unit may further be configured to alternatingly provide the inner shaft and the two or more spreadable parts with the electric current. The action of switching between one electrode, i.e. one of the inner shaft and the spreadable parts, and another electrode may be triggered by a predetermined time interval.

It is also possible to use more than one RFA devices according to the present invention in combination, such that each of the more than one RFA devices acts as an individual electrode. In this case, the RFA system may comprise two or more RFA devices and each of the RFA devices may be connected to the electric power source. The control unit may be configured for controlling an electric current provided by the electric power source between the inner shaft and/or one or more of the spreadable parts of one RFA device and the inner shaft and/or one or more of the spreadable parts of one or more other RFA devices. This technique may be advantageously used for treating dysfunctional tissues of a large extension or volume.

According to preferred embodiments of the invention, the RFA device may further comprise one or more temperature sensors respectively provided at the inner shaft and/or at the spreadable parts and the control unit may be functionally connected to the one or more temperature sensors. The temperature sensors may be configured to provide temperature information about a temperature of the inner shaft and/or of the spreadable parts and about a temperature of a tissue or part of the body of a patient surrounding the inner shaft and the spreadable parts. The control unit may further be configured for stopping the electric current provided by the electric power source from being provided to one of the inner shaft and the spreadable parts according to the temperature information obtained from the temperature sensors. Further, the control unit may be configured for starting supplying the electric current provided by the electric power source to another one of the inner shaft and the spreadable parts according to the temperature information obtained from the temperature sensors. For example, the control unit may be configured to stop providing the electric current to one of the inner shaft and the spreadable parts and to start providing the electric current to another one of the inner shaft and the spreadable parts when the temperature sensor corresponding to said one of the inner shaft and the spreadable parts measures temperature information corresponding to a predetermined temperature threshold.

In preferred embodiments of the invention, the RFA device may further comprise a fluid connector connected or connectable with a fluid supply. The fluid supply may comprise a pumping device. The inner shaft may then comprise an inner fluid lumen fluidly connecting the ablation end with the fluid connector. Additionally or alternatively, one or more of the spreadable parts may comprise a spreadable part fluid lumen fluidly connecting the respective tip with the fluid connector and/or with the inner fluid lumen. The RFA system may further comprise a fluid reservoir connected to the fluid supply, wherein the fluid supply is configured for driving a fluid flow between the fluid reservoir and the RFA device through the fluid connector. The control unit may further be configured for controlling the fluid supply for controlling the fluid flow through the fluid connector. A fluid flow "between the fluid reservoir and the fluid connector" refers herein to either one of a fluid flow from the fluid reservoir to the RFA device through the fluid connector or from the RFA device to the fluid reservoir through the fluid connector. The fluid connector, the fluid reservoir, and the fluid supply permit a flow of a fluid, like for example blood, an infusion, or chemicals, for example acids or chemotherapeutic agents, through a tissue being ablated by means of the RFA device. The fluid supply may be configured to provide a continuous or a pulsed fluid flow.

According to preferred embodiments of the invention, the control unit may be configured for automatically controlling the electric power source and/or the fluid supply. For example, the control unit may be configured for measuring and storing values or information of the electric current, parameters of the fluid flow, and/or temperature information provided by the temperature sensors and for automatically controlling the electric power source and/or the fluid supply according to predefined electric current thresholds, predefined temperature thresholds, and/or predefined fluid flow thresholds. For instance, the control unit may be configured to automatically operate the electric power source to stop providing the electric current supplied by the electric power source to one of the inner shaft and the spreadable parts and to provide it instead to another one of the inner shaft and the spreadable parts when the electric current information corresponding to said one of the inner shaft and the spreadable parts increases beyond or decreases below a predetermined threshold value. Similarly, the aforesaid predetermined threshold values may trigger the control unit to change the parameters defining a fluid flow driven by the fluid supply.

According to preferred embodiments of the invention, the RFA system may further comprise a control interface functionally connected to the control unit and configured for receiving and transmitting control instructions for controlling the control unit and/or for displaying control information from the control unit. The control information may comprise any of temperature information obtained by the temperature sensors, information about an electric current provided by the electric power source, and information about a fluid flow driven by the fluid supply. The control interface may allow a human operator controlling the RFA system through the control unit and to obtain information about the operation conditions of the RFA system. The control interface may comprise a touch screen, a display device, an input device, or the like.

In some preferred embodiments of the invention, the control interface may be wirelessly connected with the control unit.

In preferred embodiments of the invention, the control interface may comprise a sterile container. All physical components of the control interface other than the sterile container itself may be received within the sterile container.

According to preferred embodiments of the invention, the control interface may comprise a software code which, when executed on a processor, contains instructions for receiving and transmitting control instructions for controlling the control unit and/or for displaying control information from the control unit. For example, the control interface may take the form of a computer software tool or an app for a mobile device.

A further aspect of the invention relates to a method of radiofrequency ablation (RFA) treatment of a subject using a RFA device or a RFA system according to any of the embodiments described above comprising the steps of:
- inserting the RFA device into the body of the subject;
- moving the outer shaft along the longitudinal direction with respect to the inner shaft such that the two or more spreadable parts spread apart radially; and
- providing an electric current to the RFA device that flows through at least one of the inner shaft and the spreadable parts and through the body of the subject.

### BRIEF SUMMARY OF THE FIGURES

- Fig. 1: shows a schematic view of a RFA device according to an embodiment of the invention wherein the spreadable parts are spread apart from the inner shaft radially.
- Fig. 2: shows a schematic view of the RFA device of Fig. 1 in the collapsed position.
- Fig. 3: shows a schematic view of the inner shaft of the RFA device of Fig. 1 and 2.
- Fig. 4: shows a schematic view of the outer shaft of the RFA device of Fig. 1 and 2.
- Fig. 5: shows a schematic view of a RFA device according to an embodiment of the invention.
- Fig. 6: shows schematic views of cross-sections of RFA devices according to embodiments of the invention.
- Fig. 7: shows a schematic view of a RFA system according to an embodiment of the invention.
- Fig. 8: shows a flow diagram illustrating a method according to an embodiment of the invention.

### DESCRIPTION OF PREFERRED EMBODIMENTS

Certain embodiments of the present invention are described in detail herein below with reference to the accompanying drawings, wherein the features of the embodiments can be freely combined with each other unless otherwise described. However, it is to be expressly understood that the description of certain embodiments is given by way of example only, and that it should not be understood to limit the invention.

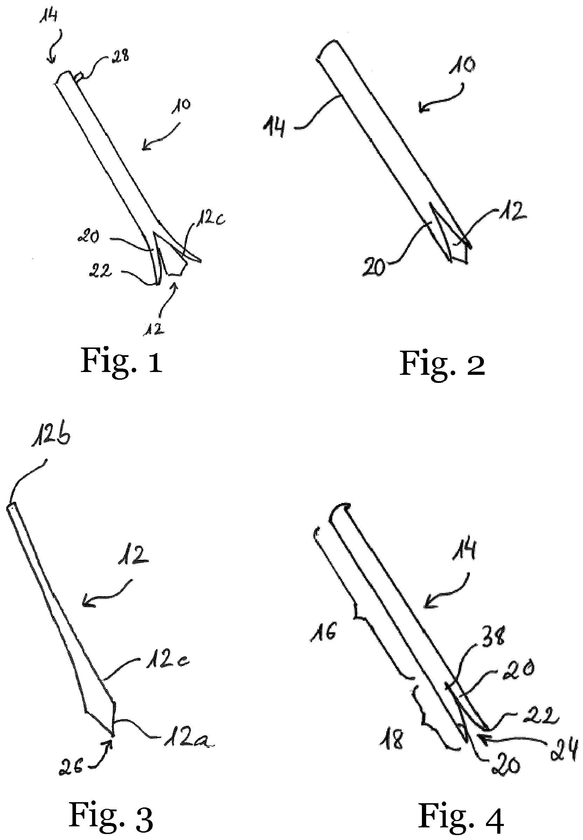

Figures 1 and 2 show schematic views of a RFA device 10 according to an embodiment of the invention. The RFA device 10 comprises an inner shaft 12, which is shown in detail in Fig. 3, that extends along the longitudinal direction between a connection end 12b and an ablation end 12a. The inner shaft 12 further comprises, in the embodiment shown, a first tip 26 at the ablation end 12a. The RFA device 10 further comprises an outer shaft 14 that also extends along the longitudinal direction. A detailed view of the outer shaft 14 is shown in Fig. 4. The outer shaft 14 has a first portion 16 and a second portion 18. In the embodiment shown, the second portion 18 comprises two spreadable parts 20 that extend between the first portion 16 and a respective tip 22 of the spreadable part 20. The two spreadable parts 20 of the outer shaft 14 are separated by two gaps 38 that are formed in the outer shaft 14 between the spreadable parts 20.

The outer shaft 14 comprises a hollow lumen 24. The hollow lumen 24 extends along the longitudinal direction and is suitable for receiving the inner shaft 12 so that the inner shaft 12 or at least a part thereof is contained within the outer shaft 14. When the inner shaft 12 is received within the outer shaft 14, the first portion 16 of the outer shaft 14 is closer to the connection end 12b than the second portion 18 and the second portion 18 of the outer shaft 14 is closer to the ablation end 12a than the first portion 16.

The situation shown in Fig. 2 corresponds to a collapsed position of the outer shaft 14 of the RFA device 10, in which the spreadable parts 20 are substantially flush with an outermost surface of the outer shaft at the first portion 16. In this collapsed position, the RFA device 10 can be easily incised into the body of a subject to provide RFA treatment.

The outer shaft 14 is movable along the longitudinal direction with respect to the inner shaft 12. By moving the outer shaft 14 along the longitudinal direction with respect to the inner shaft 12 the portion of the inner shaft 12 that is received within the outer shaft 14 can be increased or decreased. As shown in Fig. 3, the inner shaft 12 has a cross-section perpendicular to the longitudinal direction that increases along the longitudinal direction towards the ablation end 12a at least for a part of the length of the inner shaft 12. Consequently, when the inner shaft 12 is received within the outer shaft 14 and the outer shaft 14 is moved with respect the inner shaft 12 along the longitudinal direction towards the ablation end 12a, an outermost surface 12c of the inner shaft 12 exerts a radial force upon the spreadable parts 20 of the outer shaft 14 causing the spreadable parts 20 to bend outwards and to thereby spread apart radially. This corresponds to the situation shown in Fig. 1. In this situation, the distance between the spreadable parts 20 and a central axis of the outer shaft 14 is greater than in the collapsed position.

As is clear to the skilled person, the outer shaft 14 may be moved with respect to the inner shaft 12 actively or passively, that is, by moving the outer shaft 14 while the inner shaft 12 remains at rest with respect to the patient or by moving the inner shaft 12 while the outer shaft 14 remains at rest with respect to the patient.

The RFA device 10 further comprises an electric current connector 28 that is connectable to an outer electric power source. The electric current connector 28 is electrically connected with the inner shaft 12 and each of the spreadable parts 20, so that an electric current provided to the RFA device 10 through the electric current connector 28 can reach the inner shaft 12 and each of the spreadable parts 20.

In order to deliver RFA treatment to a patient, the RFA device 10 can be introduced into the patient's body through an incision while being in the collapsed position illustrated in Fig. 2. Once the ablation end 12a of the inner shaft is in the vicinity or inside of a dysfunctional tissue to be treated, like e.g. a tumour, the outer shaft 14 can be moved along the longitudinal direction with respect to the inner shaft 12 towards the ablation end 12a, thereby causing the spreadable parts 22 to spread apart radially. Since each of the inner shaft 12 and the spreadable parts 20 are electrically connected to the electric current connector 28, each of the inner shaft 12 and the spreadable parts 20 can be used as electrodes to supply RFA treatment to the tissue to be treated. When the RFA device 10 has to be moved within the patient's body or extracted therefrom, the outer shaft 14 can be moved along the longitudinal direction with respect to the inner shaft 12 towards the connection end 12b back to the collapsed position.

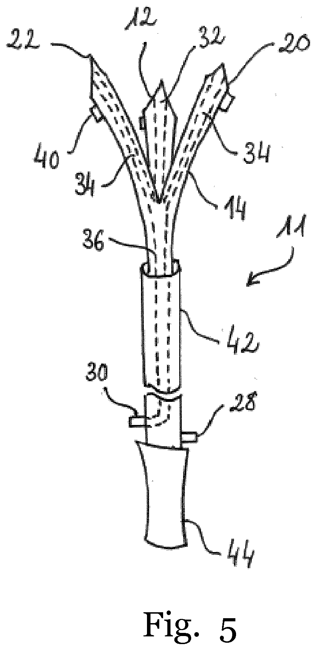

Fig. 5 shows a schematic view of an RFA device 11 according to another embodiment of the invention. The components of the RFA device 11 already described for the RFA device 10 shown in figs. 1 to 4 are indicated with the same reference signs and shall not be described again. The RFA device 11 comprises a fluid connector 30 connectable with an external fluid reservoir. The inner shaft 12 comprises an inner fluid lumen 32 that fluidly connects the ablation end 12a of the inner shaft 12 with the fluid connector 30. The inner fluid lumen 32 forms an opening at the ablation end 12a that allows establishing fluid communication between an environment of the ablation end 12 a and the fluid connector 30 through the inner fluid lumen 32. Further, each of the spreadable parts 20 of the outer shaft 14 comprises a spreadable part fluid lumen 34 that fluidly connects the respective tip 22 with the fluid connector 30 and with the inner fluid lumen 32. The inner fluid lumen 32 and the spreadable part fluid lumens 34 join to form a common fluid lumen 36 that fluidly connects each of the inner fluid lumen 32 and the spreadable part fluid lumens 34 with the fluid connector 30. Each of the spreadable part fluid lumens 34 forms an opening at the respective tip 22 that allows establishing fluid communication between an environment of said tip 22 and the fluid connector 30 through the spreadable part fluid lumen 34 and the common fluid lumen 36.

A fluid can be made to flow through the fluid connector 30, the inner fluid lumen 32 and/or the spreadable part fluid lumens 34 into the body of a subject being treated with the RFA device 10. Further, a fluid can also be made to flow out from the body of the subject through the fluid connector 30.

The RFA device 11 further comprises temperature sensors 40 that are provided at the ablation end 12a of the inner shaft 12 and at each of the spreadable parts 20 of the outer shaft 14.

The RFA device 11 also comprises a hollow insulating sleeve 42 of a polyamide material that partly encloses the inner shaft 12 and the outer shaft 14. The inner shaft 12 and the outer shaft 14 are partly received in the insulating sleeve 42. Further, the RFA device 11 comprises a control grip 44 attached to the inner shaft 12 and to the outer shaft 14 close to the connection end 12b of the inner shaft 12 and to the second portion 18 of the outer shaft 14. The control grip 44 is configured for controlling a movement of the outer shaft 14 along the longitudinal direction with respect to the inner shaft 12a and for allowing a human or a machine operator operating the RFA device 11. The outer shaft 14 and the inner shaft 12 can be moved with respect to each other along the longitudinal direction by operating the control grip 44. The RFA device 11 can be moved and rotated by holding it through the control grip 44.

In the embodiment shown in Fig. 5, the inner shaft 12 and the outer shaft 14 are made of surgical steel. When the spreadable parts 20 are spread apart, the tips 22 of the spreadable parts 20 protrude from the ablation end 12a along the longitudinal direction by 30 mm. The length of the RFA device 11 in the collapsed position is, in the embodiment shown, of 140 mm.

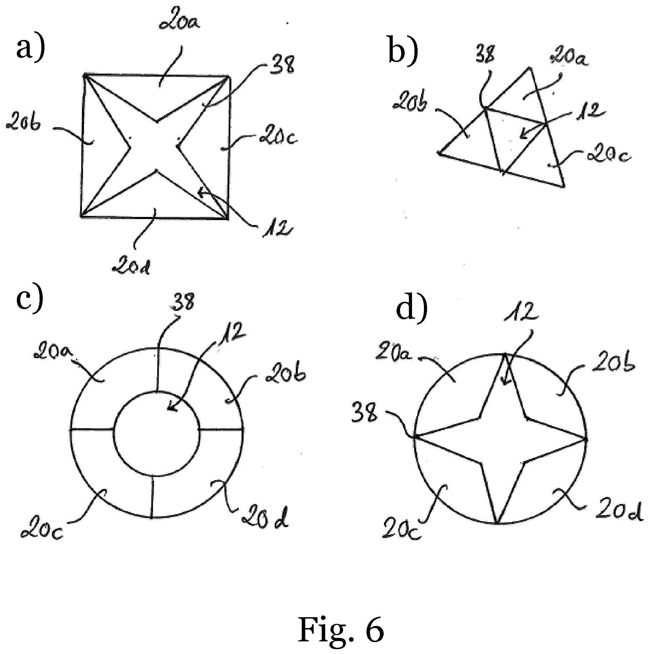

Fig. 6 shows schematic views of cross-sections of an RFA device according to an embodiment of the invention. The inner shaft 12 and the outer shaft 14 can each have a circular, a triangular, or a polygonal cross-section. Further, the outer shaft 14 may comprise a different number of spreadable parts 20 in different embodiments of the invention. In each case, the spreadable parts 20 are separated from each other by corresponding gaps 38 or slits 38 formed in the outer shaft 14. The number of gaps 38 or slits 38 corresponds to the number of spreadable parts 20 of the outer shaft 14.

In Fig. 6a, the inner shaft 12 and the outer shaft 14 have a quadrangular cross-section and the outer shaft 14 comprises four spreadable parts 20a-20d, wherein each of the spreadable parts 20a-20d extends over one of the four outer surfaces of the inner shaft 12. In the embodiment shown in Fig. 6b, the inner shaft 12 and the outer shaft 14 have a triangular cross-section and the outer shaft 14 comprises three spreadable parts 20a-20c, each extending over one of the three outer surfaces of the inner shaft 12. In the embodiment shown in Fig. 6c, the inner shaft 12 and the outer shaft 14 have a circular cross-section and the outer shaft 14 comprises four spreadable parts 20a-20d, wherein each of the spreadable parts 20a-20d extends over a portion of the outer surface of the cylindrical inner shaft 12 corresponding to an azimuthal angle of 90°. In the embodiment shown in Fig. 6d, the inner shaft 12 has a polygonal cross-section and the outer shaft 14 has a circular cross-section. The outer shaft 14 comprises four spreadable parts 20a-20d each extending over two adjacent external surfaces of the polygonal inner shaft 12.

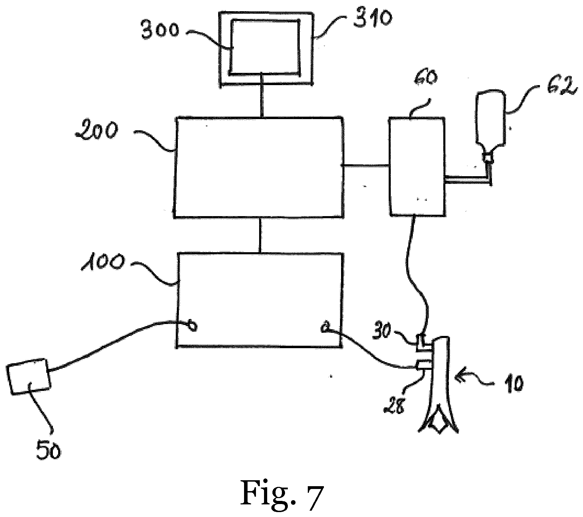

Fig. 7 shows a schematic view of an RFA system according to an embodiment of the invention. The RFA system comprises an RFA device 10 according to an embodiment of the invention, an electric power source 100, and outer electrode 50, a control unit 200, a fluid pumping device 60, and a control interface 300.

The electric power source 100 is electrically connected with the electric current connector 28 of the RFA device 10 and with the outer electrode 50. The electric current connector 28 of the RFA device 10 is electrically connected with the inner shaft 12 and each of the spreadable parts 20 of the RFA device 10. In order to supply RFA treatment to a subject, the outer electrode 50 is attached to the subject's body, the RFA device 10 is inserted into a region of the subject's body in which a dysfunctional tissue to be treated is located, and the electric power source 100 creates an electric current that circulates between the outer electrode 50 and the RFA device 10 through the dysfunctional tissue to be treated, be it through the inner shaft 12 and/or through one or more of the spreadable parts 20.

The electric power source 100 is connected to the control unit 200. The control unit 200 is configured for controlling the electric current provided by the electric power source 100 between one or more of the inner shaft 12 and the spreadable parts 20 of the RFA device 10 and the outer electrode 50. The control unit 200 can control the strength of the electric current provided by the electric power source 100. Further, the control unit 200 is configured for selectively providing the electric current to one or more of the inner shaft 12 and the spreadable parts 20 of the RFA device 10. The control unit 200 can be configured for alternatingly directing the electric current provided by the electric power source 100 to one of the inner shaft 12 and the spreadable parts 20 of the RFA device 10.

The RFA system further comprises a fluid pumping device 60 and a fluid reservoir 62, wherein the fluid reservoir 62 is fluidly connected with the fluid pumping device 60 and the fluid pumping device 60 is fluidly connected with the fluid connector 30 of the RFA device 10. The fluid pumping device 60 is configured for driving a fluid flow between the fluid reservoir 62 and the fluid connector 30. The control unit 200 is connected to the fluid pumping device 60 and configured for controlling the fluid flow between the fluid reservoir 62 and the fluid connector 30 driven by the fluid pumping device 60.

The control unit 200 can also be functionally connected to temperature sensors 40 of the RFA device 10 and control the electric power source 100 and/or the fluid pumping device 60 according to information provided by the temperature sensors 40. The connection between the control unit 200 and the temperature sensors 40 of the RFA device 10 can be a connection through the electric current connector 28 parallel to a connection through which the electric current from the electric power source 100 is provided to the RFA device 10.

The RFA system further comprises a control interface 300 that is functionally connected to the control unit 200. The control interface 300 is configured for receiving and transmitting control instructions for controlling the control unit 200 and for displaying control information from the control unit 200. For example, the control interface may display temperature values obtained by the control unit 200 from the temperatures sensors 40 of the RFA device 10 and electric current values of the electric current flowing between the RFA device 10 and the outer electrode 50 obtained by the control unit 200. Further, the control interface 300 may be configured such that a human operator can input or modify values of the temperature and the electric current used by the control unit 200 for controlling the operation of the RFA system and threshold values use by the control unit 200 to automatically control the operation of the RFA device 10. The control interface 300 can be wirelessly connected with the control unit 200. In the embodiment shown, the control interface 300 comprises a sterile container 310 that encloses all other components of the control interface 300.

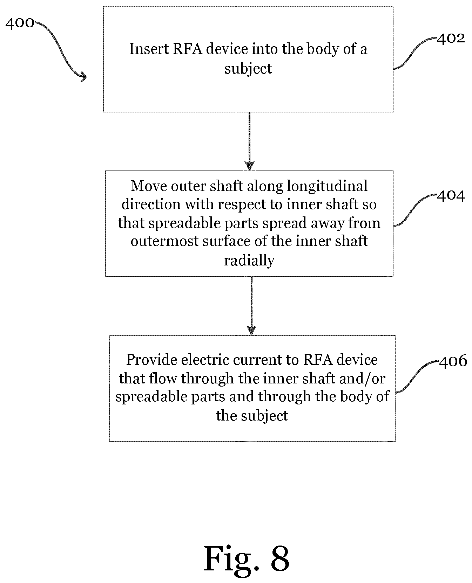

Fig. 8 shows a flow diagram illustrating a method 400 of radiofrequency ablation treatment of a subject according to an embodiment of the invention using a RFA device or a RFA system according to any of the embodiments described above.

In a first method step 402, an RFA device according to any of the embodiments of the invention described above is inserted into the body of the subject at a part thereof where a dysfunctional tissue, like e.g. a tumour, is located.

In a second method step 404, the outer shaft of the RFA device is moved along the longitudinal direction with respect to the inner shaft so that the two or more spreadable parts of the outer shaft spread apart radially as described above.

In a third method step 406, an electric current is provided to the RFA device that flows through at least one of the inner shaft and the spreadable parts and through the body of the subject, thereby ablating the dysfunctional tissue or tumour.

It is to be understood that what is described above is what is presently considered the preferred embodiment of the present invention. However, it should be noted that the description of the preferred embodiments is given by way of example only and that various modifications may be made without departing from the scope of the invention as defined in the claims.

### REFERENCE SIGN LIST

- 10, 11: RFA device
- 12: inner shaft
- 12a: ablation and
- 12b: connection and
- 12c: outer surface of the inner shaft
- 14: outer shaft
- 16: first portion of the outer shaft
- 18: second portion of the outer shaft
- 20: spreadable parts
- 20a-20d: spreadable parts
- 22: tips of the spreadable parts
- 24: hollow lumen
- 26: first tip of the inner shaft
- 28: electric current connector
- 30: fluid connector
- 32: inner fluid lumen
- 34: spreadable part fluid lumen
- 36: common lumen
- 38: gaps or slits
- 40: temperature sensors
- 42: insulating sleeve
- 44: control grip
- 50: outer electrode
- 60: fluid supply, fluid pumping device
- 62: fluid reservoir
- 100: electric power source
- 200: control unit
- 300: control interface
- 310: sterile container
- 400: method
- 402-406: method steps

## Claims

1. A radiofrequency ablation (RFA) device (10) comprising:
an inner shaft (12) extending along a longitudinal direction between a connection end (12b) and an ablation end (12a);
an outer shaft (14) extending along the longitudinal direction and having a hollow lumen (24) extending along the longitudinal direction, wherein the inner shaft (12) is received inside the hollow lumen (24);
wherein the outer shaft (14) is movable along the longitudinal direction with respect to the inner shaft (12);
wherein the outer shaft (14) comprises a first portion (16) and a second portion (18), wherein the second portion (18) is closer to the ablation end (12a) than the first portion (16), wherein the second portion (18) of the outer shaft (14) comprises two or more spreadable parts (20) extending between the first portion (16) of the outer shaft (14) and a respective tip (22) of the spreadable part (20);
wherein the inner shaft (12) has a cross-section perpendicular to the longitudinal direction that increases along the longitudinal direction towards the ablation end (12a), so that the two or more spreadable parts (20) can be made to progressively spread apart radially by moving the outer shaft (14) with respect to the inner shaft (12) along the longitudinal direction towards the ablation end (12a); and
an electric current connector (28) electrically connected or connectable with an outer electric power source;
wherein at least one of the inner shaft (12) and the spreadable parts (20) is electrically connected with the electric current connector (28).

2. The RFA device of claim 1, wherein two or more of the inner shaft (12) and the spreadable parts (20) are electrically connected with the electric current connector (28).

3. The RFA device of claim 1 or 2, wherein the inner shaft (12) and each of the spreadable parts (20) are electrically connected with the electric current connector (28).

4. The RFA device of one of the preceding claims, wherein the RFA device (10) further comprises a fluid connector (30) connected or connectable with a fluid supply;
wherein the inner shaft (12) comprises an inner fluid lumen (32) fluidly connecting the ablation end (12a) with the fluid connector (30); and/or
wherein one or more of the spreadable parts (20) comprises a spreadable part fluid lumen (34) fluidly connecting the respective tip (22) with the fluid connector (30) and/or with the inner fluid lumen (32).

5. The RFA device of one of the preceding claims, wherein the RFA device (10) further comprises one or more temperature sensors (40) respectively provided at the inner shaft (12) and/or at the spreadable parts (20); and/or
wherein the RFA device (10) further comprises a hollow insulating sleeve (42) partly enclosing the inner shaft (12) and the outer shaft (14), so that the inner shaft (12) and the outer shaft (14) are partly received in the insulating sleeve (42).

6. The RFA device of one of the preceding claims, wherein the inner shaft (12) has a circular, a triangular or a polygonal cross-section; and/or
wherein the outer shaft (14) has a circular, a triangular or a polygonal cross-section; and/or
wherein the inner shaft (12) and/or the outer shaft (14) are made of carbon, of a metallic material, preferably of stainless steel, of titanium or a of compound thereof, or of a plastic material, preferably of a polycarbonate resin, or of a thermoplastic elastomer, or of a combination thereof; and/or
wherein the inner shaft (12) comprises a first tip (26) at the ablation end (12a).

7. The RFA device of one of the preceding claims, wherein a length of the outer shaft (14) along the longitudinal direction exceeds a length of the inner shaft (12) along the longitudinal direction by between 10 mm and 80 mm, preferably by between 20 and 60 mm, more preferably by between 25 and 50 mm; and/or
wherein a length of the RFA device (10) is between 100 mm and 250 mm, preferably between 120 mm and 230 mm, more preferably between 140 and 210 mm;.

8. The RFA device of one of the preceding claims, wherein the RFA device (10) further comprises a control grip (44) operatively coupled to the inner shaft (12) and the outer shaft (14), wherein the control grip is configured for controlling a movement of the outer shaft (14) along the longitudinal direction with respect to the inner shaft (12).

9. A radiofrequency ablation (RFA) system comprising:
the RFA device (10) according to one of claims 1 to 8;
an electric power source (100) connected to the electric current connector (28); and
a control unit (200) configured for controlling an electric current provided by the electric power source (100).

10. The RFA system of claim 9, wherein two or more of the inner shaft (12) and the spreadable parts (20) of the RFA device (10) are electrically connected with the electric current connector (28); and
wherein the control unit (200) is further configured for controlling the electric current provided by the electric power source (100) to circulate between the two or more of the inner shaft (12) and the spreadable parts (20).

11. The RFA system of claim 9, further comprising at least one outer electrode (50), wherein the electric power source (100) is further connected to the at least one outer electrode (50), and wherein the control unit (200) is further configured for controlling the electric current provided by the electric power source (100) to circulate between the at least one of the inner shaft (12) and the spreadable parts (20) and the at least one outer electrode (50).

12. The RFA system of one of claims 9 to 11, wherein the control unit (200) is configured for controlling a strength of electric current provided by the electric power source (100); and/or
wherein the control unit (200) and the RFA device (10) are configured such that the control unit (200) can selectively provide the electric current to one or more of the inner shaft (12) and the spreadable parts (20); and/or
wherein the control unit (200) is configured for automatically controlling the electric power source (100) and/or the fluid supply (60).

13. The RFA system of one of claims 9 to 12, wherein the RFA device (10) further comprises one or more temperature sensors (40) respectively provided at the inner shaft (12) and/or at the spreadable parts (20); and
wherein the control unit (200) is functionally connected to the one or more temperature sensors (40).

14. The RFA system of one of claims 9 to 13, wherein the RFA device (10) further comprises a fluid connector (30) connected or connectable with a fluid supply (60);
wherein the inner shaft (12) comprises an inner fluid lumen (32) fluidly connecting the ablation end (12a) with the fluid connector (30); and/or
wherein one or more of the spreadable parts (20) comprise a spreadable part fluid lumen (34) fluidly connecting the respective tip (22) with the fluid connector (30) and/or with the inner fluid lumen (32);
wherein the RFA system further comprises a fluid reservoir (62) connected to the fluid supply (60), wherein the fluid supply (60) is configured for driving a fluid flow between the fluid reservoir (62) and the RFA device (10) through fluid connector (30); and wherein the control unit (200) is further configured for controlling the fluid supply (60) for controlling the fluid flow through the fluid connector (30).

15. The RFA system of one of claims 9 to 14, wherein the RFA system further comprises a control interface (300) functionally connected to the control unit (200) and configured for receiving and transmitting control instructions for controlling the control unit (200) and/or for displaying control information from the control unit (200);
wherein the control interface (300) preferably is wirelessly connected with the control unit (200); and/or
wherein the control interface (300) preferably comprises a sterile container (310); and/or wherein the control interface (300) preferably comprises a software code which, when executed on a processor, contains instructions for receiving and transmitting control instructions for controlling the control unit (200) and/or for displaying control information from the control unit (200).
